# EUROPEAN PATENT SPECIFICATION

(11) **EP 2 297 128 B1**
(45) Date of publication and mention of the grant of the patent: **15.08.2012**
(21) Application number: 08737089.6
(22) Date of filing: 23.04.2008
(51) Int. Cl.: C07D 333/38, A61K 31/381, A61P 25/28, A61P 37/00

(54) **INHIBITORS OF IKK- SERINE-THREONINE PROTEIN KINASE**
INHIBITOREN VON IKK-SERINTHREONINPROTEINKINASE
INHIBITEURS DE SÉRINE-THRÉONINE PROTÉINE KINASE IKK-

(43) Date of publication of application: 23.03.2011
(73) Proprietor: Chroma Therapeutics Ltd., Abingdon Oxfordshire OX14 4RY (GB)
(72) Inventor: MOFFAT, David, Festus, Charles, Abingdon, Oxfordshire OX14 4RY (GB); DAVIES, Stephen, John, Abingdon, Oxfordshire OX14 4RY (GB); CHARLTON, Michael, Hugh, Abingdon, Oxfordshire OX14 4RY (GB); HIRST, Simon, Christopher, Nottingham NG1 1GF (GB); ONIONS, Stuart, Thomas, Nottingham NG1 1GF (GB); WILLIAMS, Jonathon, Gareth, Nottingham NG1 1GF (GB)
(74) Representative: Simons, Amanda Louise
(86) International application number: PCT/GB2008/001434
(87) International publication number: WO 2009/130434

(56) References cited:
- WO-A-2004/063185
- WO-A-2008/053182
- WO-A-2008/053185

## Description

This invention relates to thiophene carboxamides characterised by the presence in the molecule of an amino acid ester group, to compositions containing them, to processes for their preparation and to their use in medicine as IKK inhibitors for the treatment of autoimmune and inflammatory diseases, including chronic obstructive pulmonary disease, asthma, rheumatoid arthritis, psoriasis, inflammatory bowel disease, Crohn's disease, ulcerative colitis, multiple sclerosis, diabetes, atopic dermatitis, graft versus host disease, systemic lupus erythematosus. The compounds are also of use in the treatment of proliferative disease states, such as cancer.

### Background of the Invention

The expression of many pro-inflammatory genes is regulated by the transcriptional activator nuclear factor-*k*B (NF-*k*B). These transcription factors have been suspected since their discovery to play a pivotal role in chronic and acute inflammatory diseases. It now seems that aberrant regulation of NF-*k*B could also underlie autoimmune diseases and different types of cancer.

Examples of genes dependent on the activation of NF-*k*B include: the cytokines tumour necrosis factor TNF-α, interleukin (IL)-6, IL-8 and IL-1β; the adhesion molecules E-selectin, intercellular adhesion molecule (ICAM)-1 and vascular cell adhesion molecule (VCAM)-1; and the enzymes nitric oxide synthase (NOS) and cyclooxygenase (COX)-2. NF-*k*B normally resides in the cytoplasm of unstimulated cells as an inactive complex with a member of the IkB inhibitory protein family. However, upon cellular activation, IkB is phosphorylated by the IkB kinase (IKK) and is subsequently degraded. Free NF-*k*B then translocates to the nucleus where it mediates pro-inflammatory gene expression.

There are three classical I*k*B's: I*k*Bα, I*k*Bβ and I*k*Bε; all of which require the phosphorylation of two key serine residues before they can be degraded. Two major enzymes IKK-α and Irk-β appear to be responsible for I*k*B phosphorylation. Dominant-negative (DN) versions of either of these enzymes (where ATP binding is disabled by the mutation of a key kinase domain residue) were found to suppress the activation of NF-*k*B by TNF-α, IL-1β and LPS. Importantly IKK-β DN was found to be a far more potent inhibitor than IKK-α DN (Zandi, E Cell, 1997, 91, 243). Furthermore, the generation of IKK-α and IKK-β deficient mice established the requirement of IKk-β for activation of NF-*k*B by proinflammatory stimuli and reinforced the dominant role of Irk-β suggested by biochemical data. Indeed it was demonstrated that IKK-α was dispensable for NF-*k*B activation by these stimuli (Tanaka, M.; Immunity 1999, 10, 421). Thus, inhibition of IKKβ represents a potentially attractive target for modulation of immune function and hence the development of drugs for the treatment of auto-immune diseases.

Our co-pending International Patent Application No: PCT/GB2007/004117 relates to IKKβ inhibitors of formula (IA) or (IB), or a salt, N-oxide, hydrate or solvate thereof: 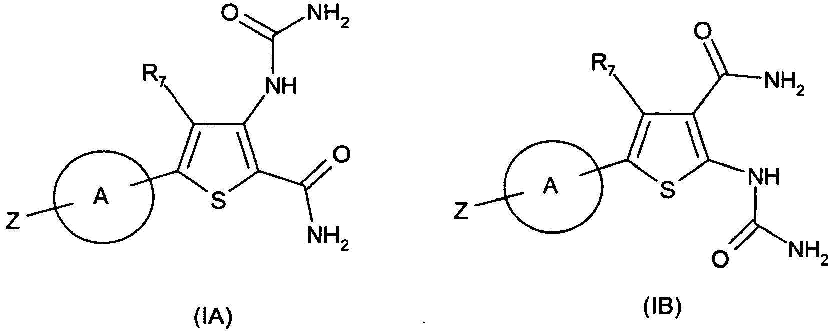 wherein
**R₇** is hydrogen or optionally substituted (C₁-C₆)alkyl;
**ring A** is an optionally substituted aryl or heteroaryl ring or ring system of 5-13 ring atoms;
**Z** is a radical of formula **R-L¹-Y¹-(CH₂)_{z}**-, wherein:
   **R** is a radical of formula (X) or (Y) 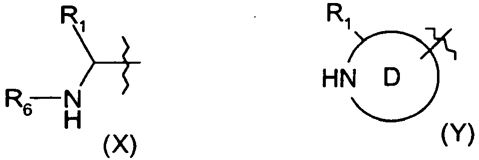
   **R₁** is a carboxylic acid group (-COOH), or an ester group which is hydrolysable by one or more intracellular esterase enzymes to a carboxylic acid group;
   **R₆** is hydrogen; or optionally substituted C₁-C₆ alkyl, C₃-C₇ cycloalkyl, aryl or heteroaryl or -(C=O)R₃, -(C=O)OR₃, or -(C=O)NR₃ wherein R₃ is hydrogen or optionally substituted (C₁-C₆)alkyl,
   **Y¹** is a bond, -(C=O)-, -S(O₂)-, -C(=O)O-, -OC(=O)-, -(C=O)NR₃-, -NR₃(C=O)-, -S(O₂)NR₃-, -NR₃S(O₂)-, or -NR₃(C=O)NR₄-, wherein R₃ and R₄ are independently hydrogen or optionally substituted (C₁-C₆)alkyl,
   **L¹** is a divalent radical of formula -(Alk¹)ₘ(Q)ₙ(Alk²)p- wherein
      **m, n** and **p** are independently 0 or 1,
      **Q** is (i) an optionally substituted divalent mono- or bicyclic carbocyclic or heterocyclic radical having 5-13 ring members, or (ii), in the case where p is 0, a divalent radical of formula -Q¹-X²- wherein X² is -O-, - S- or NR^{A}- wherein R^{A} is hydrogen or optionally substituted C₁-C₃ alkyl, and Q¹ is an optionally substituted divalent mono- or bicyclic carbocyclic or heterocyclic radical having 5-13 ring members,
      **Alk¹** and **Alk²** independently represent optionally substituted divalent C₃-C₇ cycloalkyl radicals, or optionally substituted straight or branched, C₁-C₆ alkylene, C₂-C₆ alkenylene, or C₂-C₆ alkynylene radicals which may optionally contain or terminate in an ether (-O-), thioether (-S-) or amino (-NR^{A}-) link wherein R^{A} is hydrogen or optionally substituted C₁-C₃ alkyl; and
   **z** is 0 or 1.

The compounds (IA) and (IB) above have an alpha amino acid or alpha amino acid ester motif R, linked through the alpha carbon to the main molecular template via a linker radical -L¹-Y¹-(CH₂)_{Z}-. The C-linked amino acid ester motif promotes transport across cell membranes into the cell, where intracellular esterases hydrolyse the ester to the parent acid. This charged species is poorly transported back across the cell membrane, leading to accumulation of IKKβ inhibitor activity in the cell.

WO2004/063185 discloses thiophene carboxamides which are inhibitor of IKKβ.

Our co-pending International Patent Application No: PCT/GB2007/004114 relates to IKKβ inhibitors of formula (IA1) or (IB1), or a salt, N-oxide, hydrate or solvate thereof: 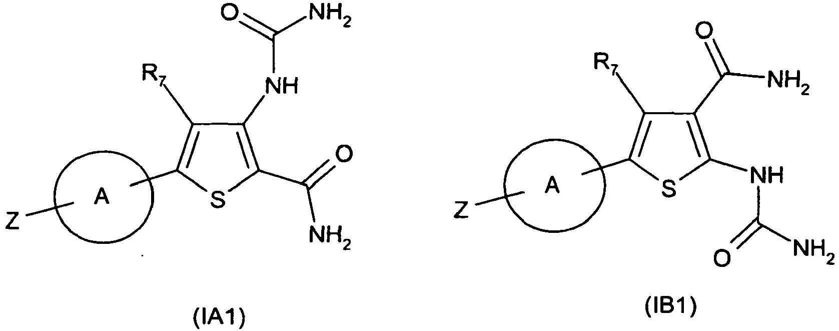 wherein
**R₇** is hydrogen or optionally substituted (C₁-C₆)alkyl;
ring **A** is an optionally substituted aryl or heteroaryl ring or ring system of 5-13 ring atoms;
**Z** is (a) a radical of formula **R₁R₂CHNH-Y-L¹-X¹-(CH₂)_{z}**- wherein:
   **R₁** is a carboxylic acid group (-COOH), or an ester group which is hydrolysable by one or more intracellular esterase enzymes to a carboxylic acid group;
   **R₂** is the side chain of a natural or non-natural alpha amino acid;
   **Y** is a bond, -C(=O)-, -S(=O)₂-, -C(=O)O-, -C(=O)NR₃-, -C(=S)-NR₃, -C(=NH)-NR₃ or -S(=O)₂NR₃- wherein R₃ is hydrogen or optionally substituted C₁-C₆ alkyl;
   **L¹** is a divalent radical of formula -(Alk¹)ₘ(Q)ₙ(Alk²)ₚ- wherein
      **m, n** and **p** are independently 0 or 1,
      **Q** is (i) an optionally substituted divalent mono- or bicyclic carbocyclic or heterocyclic radical having 5-13 ring members, or (ii), in the case where p is 0, a divalent radical of formula -Q¹-X²- wherein X² is -O-, - S- or NR^{A}- wherein R^{A} is hydrogen or optionally substituted C₁-C₃ alkyl, and Q¹ is an optionally substituted divalent mono- or bicyclic carbocyclic or heterocyclic radical having 5-13 ring members,
      **Alk¹** and **Alk²** independently represent optionally substituted divalent C₃-C₇ cycloalkyl radicals, or optionally substituted straight or branched, C₁-C₆ alkylene, C₂-C₆ alkenylene, or C₂-C₆ alkynylene radicals which may optionally contain or terminate in an ether (-O-), thioether (-S-) or amino (-NR^{A}-) link wherein R^{A} is hydrogen or optionally substituted C₁-C₃ alkyl;
   **X¹** is a bond, -C(=O)-; or -S(=O)₂-; -NR₄C(=O)-, -C(=O)NR₄-, -NR₄C(=O)-NR₅-. -NR₄S(=O)₂-, or -S(=O)₂NR₄- wherein R₄ and R₅ are independently hydrogen or optionally substituted C₁-C₆ alkyl; and
   **z** is 0 or 1.

The compounds (IA1) and (IB1) above also have an alpha amino acid or alpha amino acid ester motif R₁R₂CHNH-, this time linked thorough the alpha amino group to the main molecular template via a linker radical -Y-L¹-X¹-(CH₂)_{Z}-. The N-linked amino ester motif, like its C-linked counterpart, also promotes transport across cell membranes into the cell, where intracellular esterases hydrolyse the ester to the parent acid. This charged species is poorly transported back across the cell membrane, leading to accumulation of IKKβ inhibitor activity in the cell.

International Patent Application No: PCT/GB2007/004114 also disclosed that many of the compounds with which it is concerned and which have an alpha amino acid ester motif selectively accumulate IKK activity in macrophages. Macrophages are known to play a key role in inflammatory disorders through the release of cytokines in particular TNFα and IL-1 (van Roon et al, Arthritis and Rheumatism, 2003, 1229-1238). In rheumatoid arthritis they are major contributors to the maintenance of joint inflammation and joint destruction. Macrophages are also involved in tumour growth and development (Naldini and Carraro, Curr Drug Targets lnflamm Allergy, 2005, 3-8). Hence agents that selectively target macrophage cell proliferation could be of value in the treatment of cancer and autoimmune disease. Targeting specific cell types would be expected to lead to reduced side-effects. The way in which the esterase motif is linked to the main IKKβ inhibitor template determines whether it is hydrolysed, and hence whether or not it accumulates in different cell types. Specifically, macrophages contain the human carboxylesterase hCE-1 whereas other cell types do not. In the formulae (IA1) and (IB1) of PCT/GB2007/004114, when the nitrogen of the esterase motif R₁CH(R₂)NH- is not directly linked to a carbonyl (-C(=O)-), i.e. when Y is not a -C(=O), -C(=O)O- or -C(=O)NR₃- radical, the ester will only be hydrolysed by the intracellular carboxylesterase hCE-1, and not by the carboxylesterases hCE-2 and hCE-3. Since only cells of the monocyte/macrophage lineage have been found to contain hCE-1, the inhibitors of PCT/GB2007/004114 which meet those structural requirements will only accumulate in macrophages. Herein, unless "monocyte" or "monocytes" is specified, the term macrophage or macrophages will be used to denote macrophages (including tumour associated macrophages) and/or monocytes.

### Brief Description of the Invention

The present invention relates to a group of specific compounds falling within the general disclosures of PCT/GB2007/004117 and PCT/GB2007/004114, but not specifically identified or exemplified therein. The present compounds have the utilities disclosed in those applications, and in particular those falling within the class of PCT/GB2007/004114 which meet the structural requirements referred to in the preceding paragraph display the macrophage selectivity property discussed above.

### Detailed Description of the Invention

According to the invention there is provided a compound selected from the group consisting of:
*Cyclopentyl (2*S*,4*E*)-2-amino-5-{3-[4-carbamoyl-5 (carbamoylamino)-2-thienyl]phenyl}pent-4-enoate;
Cyclopentyl 5-{3-[4-carbamoyl-5-(carbamoylamino)-2-thienyl]phenyl}-L-norvalinate;
Cyclopentyl (2*S*,4*E*]-2-amino-5-{3-[4-carbamoyl-5-(carbamoylamino)-2-thienyl]-5-methylphenyl}pent-4-enoate;
Cyclopentyl (2*S*,4*E*)-2-amino-5-{5-[4-carbamoy)-5-(carbamoytamino)-2-thieny)]-2-methylphenyl}pent-4-enoate;
*Cyclopentyl *O*-{3-[4-carbamoyl-5-(carbamoylamino)-2-thienyl]phenyl}-L-homoserinate;
*Cyclopentyl N-{3-[4-carbamoyl-5-(carbamoylamino)-2-thieny)]benzyl}-L-alaninate;
**tert*-Butyl *N*-{3-[4-carbamoyl-5-(carbamoylamino)-2-thienyl]benzyl}-L-alaninate;
(2*S*,4*E*)-2-amino-5-{3-[4-carbamoyl-5-(carbamoylamino)-2-thienyl]phenyl}pent-4-enoic acid;
5-{3-[4-carbamoyl-5-(carbamoylamino)-2-thienyl]phenyl}-L-norvaline;
(2S,4*E*)-2-amino-5-{3-[4-carbamoyl-5-(carbamoylamino}-2-thienyl]-5-methylphenyl}pent-4-enoic acid;
(2*S*,4*E*)-2-amino-5-{5-[4-carbamoyl-5-(carbamoylamino)-2-thienyl]-2-methylphenyl}pent-4-enoic acid;
*O*-{3-[4-carbamoyl-5-(carbamoylamino)-2-thienyl]phenyl}-L-homoserine;
*N-*{3-[4-carbamoyl-5-(carbamoylamino)-2-thienyl]benzyl}-L-alanine;
and pharmaceutically acceptable salts thereof.

As used herein the term "salt" includes base addition, acid addition and quaternary salts. Compounds of the invention which are acidic can form salts, including pharmaceutically acceptable salts, with bases such as alkali metal hydroxides, e.g. sodium and potassium hydroxides; alkaline earth metal hydroxides e.g. calcium, barium and magnesium hydroxides; with organic bases e.g. N-methyl-D-glucamine, choline tris(hydroxymethyl)amino-methane, L-arginine, L-lysine, N-ethyl piperidine, dibenzylamine and the like. Those compounds (I) which are basic can form salts, including pharmaceutically acceptable salts with inorganic acids, e.g. with hydrohalic acids such as hydrochloric or hydrobromic acids, sulphuric acid, nitric acid or phosphoric acid and the like, and with organic acids e.g. with acetic, tartaric, succinic, fumaric, maleic, malic, salicylic, citric,methanesulphonic, p-toluenesulphonic, benzoic, benzenesulphonic, glutamic, lactic, and mandelic acids and the like. For a review on suitable salts, see Handbook of Pharmaceutical Salts: Properties, Selection, and Use by Stahl and Wermuth (Wiley-VCH, Weinheim, Germany, 2002).

As is common in medicinal chemistry it is expected that the compounds of the invention may be isolated in various hydrated or solvated forms, and in different crystal habits, The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

As used herein, the term "compound of the invention" refers to the 13 compounds listed above, and includes their pharmaceutically acceptable salts, hydrates and solvates.

Of the compounds of the invention, those marked with an asterisk in the list above are currently preferred.

In another broad aspect the invention provides the use of a compound of the invention in the preparation of a composition for inhibiting the activity of IKK, especially Irk-β, as well as diseases modulated by the NF-*k*B cascade. The compounds of the invention may be used for the inhibition of IKK, especially IKK-β, activity *in vitro* or *in vivo.*

Pharmaceutical compositions comprising a compound of the invention together with one or more pharmaceutically acceptable carriers and/or excipients, also form part of the invention.

As mentioned above, the compounds with which the invention is concerned are inhibitors of IKK, especially IKK-β kinase activity, and are therefore of use in the treatment of diseases modulated by IKK activity and the NF-*k*B cascade. Such diseases include neoplastic/proliferative, immune and inflammatory disease. In particular, uses of the compounds include treatment of cancers such as hepatocellular cancer or melanoma, but including bowel cancer, ovarian cancer, head and neck and cervical squamous cancers, gastric or lung cancers, anaplastic oligodendrogliomas, glioblastoma multiforme or medulloblastomas; and treatment of rheumatoid arthritis, psoriasis, inflammatory bowel disease, Crohn's disease, ulcerative colitis, chronic obstructive pulmonary disease, asthma, multiple sclerosis, diabetes, atopic dermatitis, graft versus host disease, or systemic lupus erythematosus. Other indications include metabolic disorders such as.Type II diabetes mellitus and neurological disorders such as Alzheimers disease.

The compounds with which the invention is concerned may be prepared for administration by any route consistent with their pharmacokinetic properties. The orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; nonaqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The compounds of the invention may be administered in inhaled form. Aerosol generation can be carried out using, for example, pressure-driven jet atomizers or ultrasonic atomizers, preferably using propellant-driven metered aerosols or propellant-free administration of micronized active compounds from, for example, inhalation capsules or other "dry powder" delivery systems. The active compounds may be dosed as described depending on the inhaler system used. In addition to the active compounds, the administration forms may additionally contain excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhalers for example as described EP-A-0505321).

For topical application to the eye, the drug may be made up into a solution or suspension in a suitable sterile aqueous or non aqueous vehicle. Additives, for instance buffers such as sodium metabisulphite or disodium edeate; preservatives including bactericidal and fungicidal agents such as phenyl mercuric acetate or nitrate, benzalkonium chloride or chlorhexidine, and thickening agents such as hypromellose may also be included.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agent can be dissolved in the vehicle.

The compounds of the invention may be used in conjunction with a number of known pharmaceutically active substances. For example, the compounds of the invention may be used with cytotoxics, HDAC inhibitors, kinase inhibitors, aminopeptidase inhibitors, protease inhibitors, bcl-2 antagonists, inhibitors of mTor and monoclonal antibodies (for example those directed at growth factor receptors). Preferred cytotoxics include, for example, taxanes, platins, anti-metabolites such as 5-fluoracil, topoisomerase inhibitors and the like. The medicaments of the invention comprising amino acid derivatives of formula (IA), (IB), (IA1) or (IB1), tautomers thereof or pharmaceutically acceptable salts, N-oxides, hydrates or solvates thereof therefore typically further comprise a cytotoxic, an HDAC inhibitor, a kinase inhibitor, an aminopeptidase inhibitor and/or a monoclonal antibody.

Further, the present invention provides a pharmaceutical composition comprising:
(a) a compound of the invention;
(b) a cytotoxic agent, an HDAC inhibitor, a kinase inhibitor, an aminopeptidase inhibitor, a protease inhibitor, a bcl-2 antagonist, an inhibitor of mTor and/or a monoclonal antibody; and
(c) a pharmaceutically acceptable carrier or diluent.

Also provided is a product comprising:
(a) a compound of the invention; and
(b) a cytotoxic agent, an HDAC inhibitor, a kinase inhibitor, an aminopeptidase inhibitor, a protease inhibitor, a bcl-2 antagonist, an inhibitor of mTor and/or a monoclonal antibody,
for the separate, simultaneous or sequential use in the treatment of the human or animal body.

The preparation of the compounds of the invention will now be described:

### Abbreviations

MeOH = methanol
EtOH = ethanol
IPA = isopropyl alcohol
EtOAc = ethyl acetate
DCM = dichloromethane
DMF = dimethylformamide
DME = 1,2-dimethoxy ethane
DMSO = dimethyl sulfoxide
DMAP = 4-dimethylamino pyridine
TFA = trifluoroacetic acid
THF = tetrahydrofuran
FMOC = 9-fluorenylmethoxycarbonyl
Na₂CO₃ = sodium carbonate
DIPEA = diisopropylethylamine
MP-CNBH₃ = macroporous triethylammonium methylpolystyrene cyanoborohydride
BEMP = 2-tbutylimino-2-diethylamino-1,3-dimethyl-perhydro-1,3,2-diazaphosphorine
NaH = sodium hydride
NaOH = sodium hydroxide
NaHCO₃ = sodium hydrogen carbonate
HCI = hydrochloric acid
Pd/C = palladium on carbon
PdCl₂(dppf) = [1,1'-Bis(diphenylphosphino)ferrocene) dichloropalladium(II).
EDCI = 1-ethyl-3-(3-dimethylaminopropyl)carbodiimide
KOAc = potassium acetate
TBAI = tetrabutyl ammonium iodide
ml = millilitre(s)
g = gram(s)
mg = milligram(s)
mol = mole(s)
mmol = millimole(s)
Sat = saturated
LCMS = high performance liquid chromatography/mass spectrometry
NMR = nuclear magnetic resonance
RT = room temperature

Commercially available reagents and solvents (HPLC grade) were used without further purification. Solvents were removed using a Buchi rotary evaporator. Microwave irradiation was carried out using a CEM Discovery model set at 300W. Purification of compounds by flash chromatography column was performed using silica gel, particle size 40-63µ µm (230-400 mesh) obtained from Fluorochem. Purification of compounds by preparative HPLC was performed on a Agilent prep system using reverse phase Agilent prep-C18 columns 5µm, 50 x 21.2 mm), gradient 0-100% B (A = water / 0.1 % ammonia or 0.1% formic acid and B = acetonitrile / 0.1 % ammonia or 0.1 % formic acid) over 10 min, flow = 28 ml/min, UV detection at 254 nm.

¹H NMR spectra were recorded on a Bruker 400 or 300 MHz AV spectrometer in deuterated solvents. Chemical shifts (δ) are in parts per million. Thin-layer chromatography (TLC) analysis was performed with Kieselgel 60 F₂₅₄ (Merck) plates and visualized using UV light.

Analytical HPLC/MS were obtained as follows: Agilent Prep-C18 Scalar column, 5 µm (4.6 x 50 mm, flow rate 2.5 ml/min) eluting with a H₂O-MeCN gradient containing 0.1% v/v formic acid over 7 minutes with UV detection at 254 nm. Gradient information: 0.0 - 0.5 min: 95% H₂O-5% MeCN; 0.5 -5.0 min; Ramp from 95% H₂O 5% MeCN to 5% H₂O-95% MeCN; 5.0 - 5.5 min: Hold at 5% H₂O-95% MeCN; 5.5 - 5.6 min: Hold at 5% H₂O-95% MeCN, flow rate increased to 3.5 ml/min; 5.6 - 6.6 min: Hold at 5% H₂O-95% MeCN, flow rate 3.5 ml/min; 6.6 - 6.75 min: Return to 95% H₂O-5% MeCN, flow rate 3.5 ml/min; 6.75 - 6.9 min: Hold at 95% H₂O-5% MeCN, flow rate 3.5 ml/min; 6.9 - 7.0 min: Hold at 95% H₂O-5% MeCN, flow rate reduced to 2.5 ml/min. Mass spectra were obtained using an Agilent multimode source in either the positive (APCI + ESI⁺) or negative (APCI + ESI⁻) mode.

Examples of such methods that may be employed in the synthesis of compounds of general formula (IA), (IB), (IA1) and (IB1) are set out, but not limited to the reactions shown in Schemes 1-7 below.

Scheme 1 illustrates the general synthetic route for the preparation of the examples described below, using traditional Suzuki chemistry to couple the relevant boronate ester **(Intermediate 2)** with the central thiophene core **(Intermediate 1)**.

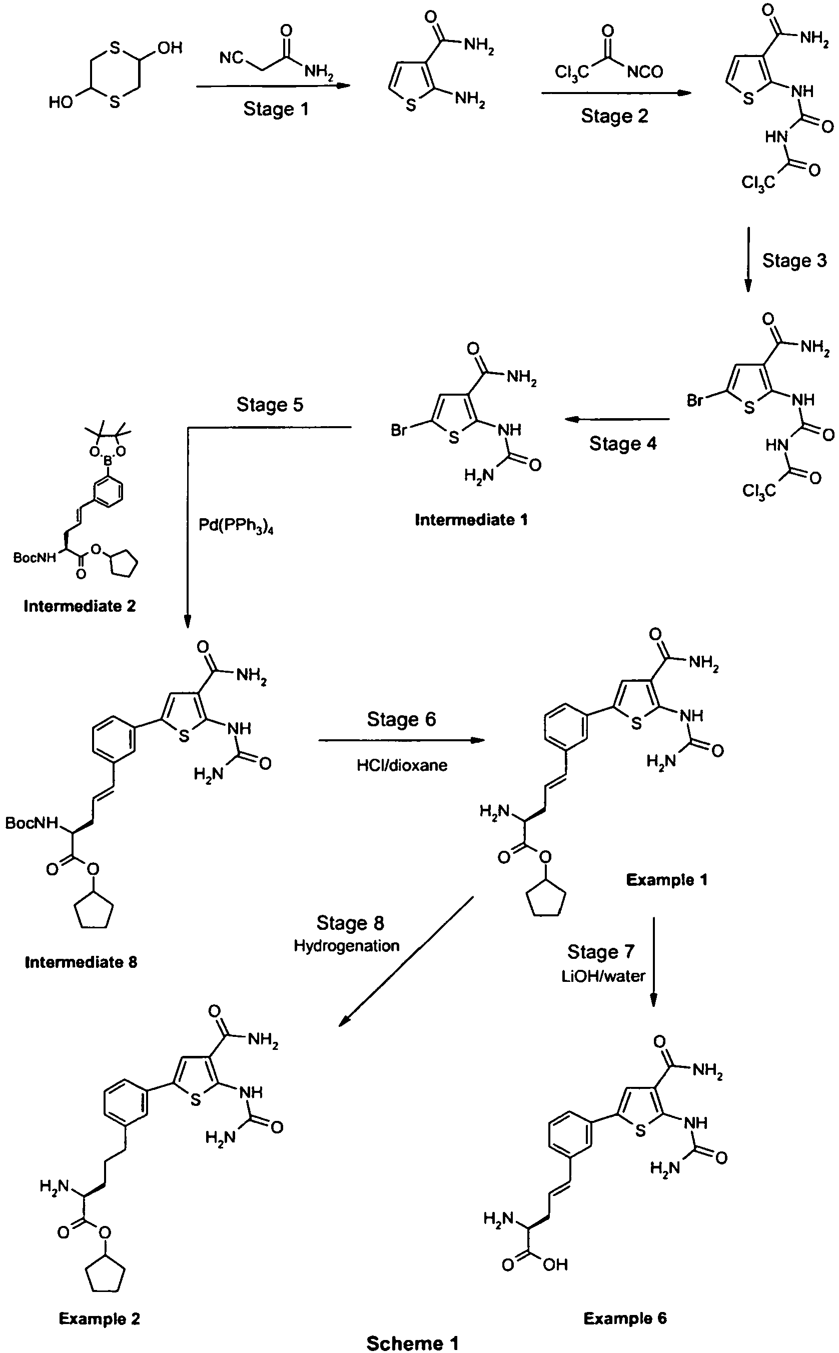

Scheme 2 illustrates the synthesis to **Intermediate 2**.

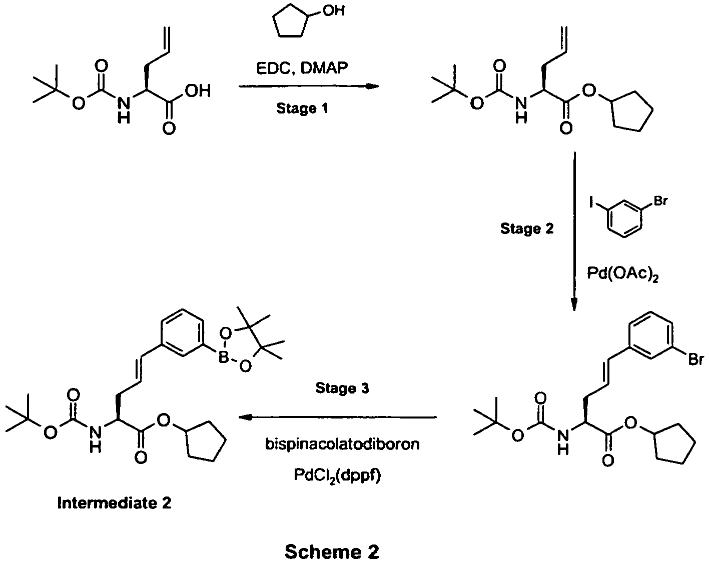

Schemes 3 and 4 illustrate the synthesis to **Intermediates 5** and **6**

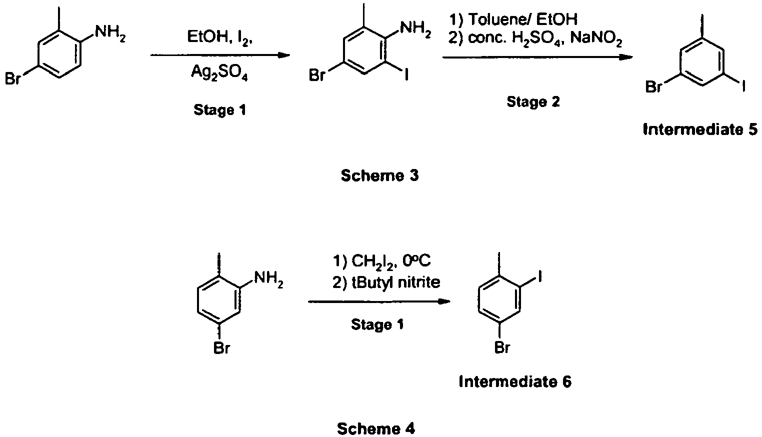

Scheme 5 illustrates the synthesis to **Intermediate 7**

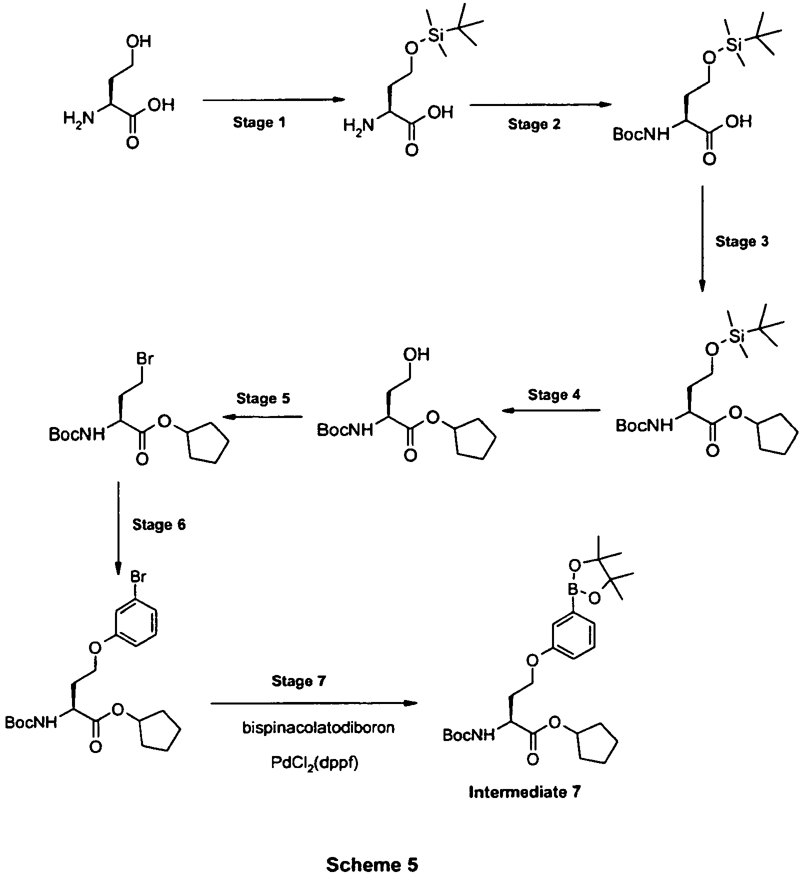

Scheme 6 illustrates the general synthetic route for the preparation of the **Examples** 11-13, using traditional Suzuki chemistry to couple the relevant boronate acid **(Intermediates 10a-10b)** with the central thiophene core **(Intermediate 1).**

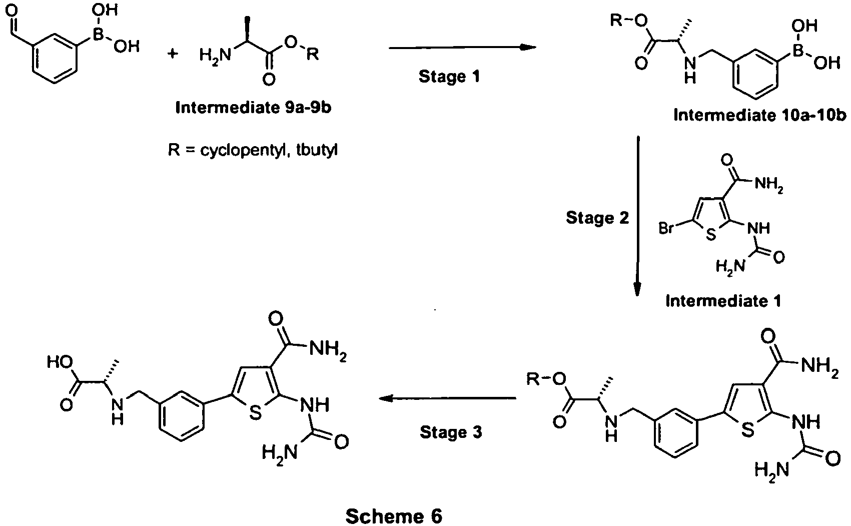

### Intermediates

### Intermediate 1: 5-Bromo-2-(carbamoylamino)thiophene-3-carboxamide

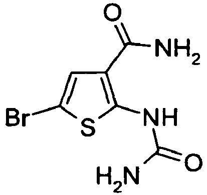

The synthesis of **Intermediate 1** described by Stages 1-4 (Scheme 1) is detailed within WO03104218.

### Intermediate 2: Cyclopentyl (2S,4E)-2-[(tert-butoxycarbonyl)aminol-5-[3-(4,4.5,5-tetramethyl-1,3,2-dioxaborolan-2-yl)phenyl]pent-4-enoate

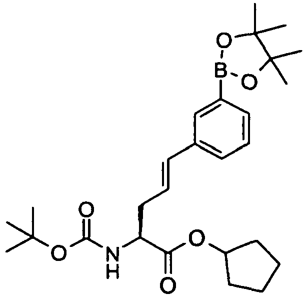

The synthesis of **Intermediate 2** is detailed within Scheme 2 and full experimental details are shown below.

### Stage 1: Cyclopentyl (2S)-2-[(tert-butoxycarbonyl)amino]pent-4-enoate

To a solution of (2*S*)-2-[(*tert*-butoxycarbonyl)amino]pent-4-enoic acid (2.00 g, 9.29 mmol) in DCM (20 ml), at 0°C, was added cyclopentanol (942 µl, 11.15 mmol) and DMAP (113 mg, 0.93 mmol). The mixture was stirred for 15 minutes, EDCI (1.99 g, 10.22 mmol) was added and the reaction stirred for 3 hrs at 0°C. The reaction was diluted with DCM, washed with H₂O brine, dried (MgSO₄) and concentrated *in vacuo.* The residue was purified by column chromatography (3-5% EtOAc in Heptane) to provide the title compound as an oil (2.15g, 82% yield). LCMS: m/z 284 [M+H]⁺.

### Stage 2- Cyclopentyl (2S,4E)-5-(3-bromophenyl)-2-[(tert-butoxycarbonyl)amino]pent-4-enoate

To a mixture of cyclopentyl (2*S*)-2-[(*tert*-butoxycarbonyl)amino]pent-4-enoate (2g, 7.06 mmol), 3-bromo-iodobenzene (1.35 ml, 10.59 mmol), NaHCO₃ (1.78 g, 21.18 mmol), TBAI (2.87 g, 7.77 mmol) in acetonitrile (15 ml) was added Pd(OAc)₂ (159 mg, 0.71 mmol). The mixture was purged with N₂ and heated for 24 hrs at 77°C. Further 3-bromo-iodobenzene (800 µl, 5.3 mmol) and Pd(OAc)₂ (159 mg, 0.71 mmol) were added and the mixture heated for 24 hrs at 77°C. The mixture was cooled, diluted with EtOAc, filtered through Celite®, washed with H₂O, brine, dried (MgSO₄) and concentrated *in vacuo.* The residue was purified by column chromatography (4-10% EtOAc in Heptane) to provide an oil (1.38 g) containing the title compound with minor quantity of cyclopentyl (2S)-2-[(*tert*-butoxycarbonyl)amino]pent-4-enoate and an unknown impurity. The crude product was used without further purification. LCMS: m/z 460. [M+H]⁺.

### Stage 3- Cyclopentyl (2S,4E)-2-[(tert-butoxycarbonyl)amino]-5-[3-(4,4,5,5-tetramethyl-1,3,2-dioxaborolan-2-yl)phenyl]pent-4-enoate

To a mixture of cyclopentyl (2*S*,4*E*)-5-(3-bromophenyl)-2-[(*tert-*butoxycarbonyl)amino] pent-4-enoate (1.38 g, 3.15 mmol), KOAc (402 mg, 4.10 mmol) and 4,4,4',4',5,5,5',5'-octamethyl-2,2'-bi-1,3,2-dioxaborolane (1.48 g, 6.29 mmol) in DMSO (7.8 ml) was added PdCl₂(dppf) (257 mg, 0.32 mmol) and the mixture purged with N₂. The reaction was heated at 65°C under N₂ for 22 hrs, cooled and partitioned between Et₂O/H₂O. The aqueous was extracted with Et₂O combined organics washed with H₂O brine, dried (MgSO₄) and concentrated. The residue was purified by column chromatography (7-10% EtOAc in Heptane) to provide the title compound (1.09 g, 71% yield). LCMS: m/z 508.2 [M+Na+H]⁺. ¹H NMR (300 MHz, CDCl₃) δ: 7.80 (1H, s), 7.68 (1H, d, J=7.5Hz), 7.44 (1H, d, J=7.5Hz), 7.36-7.30 (1H, m), 6.47 (1H, d, J=15.9Hz), 6.20-6.07 (1H, m), 5.27-5.21 (1H, m), 5.15-5.07 (1H, m), 4.45-4.36 (1H, m), 2.75-2.56 (1H, m), 1.95-1.62 (8H, m), 1.45 (9H, s), 1.37 (12H, s).

### Intermediate 3: Cyclopentyl (2S,4E)-2-[(tert-butoxycarbonyl)amino]-5-[2-methyl-5-(4,4,5,5-tetramethyl-1,3,2-dioxaborolan-2-yl)phenyl]pent-4-enoate

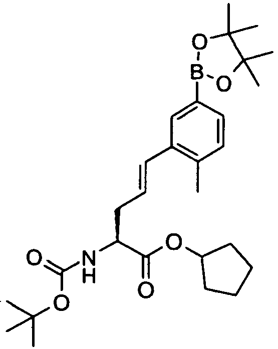

Synthesised by analogous methods to **Intermediate** 2, using **Intermediate 6** at Stage 2 of Scheme 2.
LCMS: m/z 500 [M+H]⁺. ¹H NMR (300 MHz, CDCl₃) δ: 7.84 (1H, s), 7.62-7.58 (1H, m), 7.19-7.12 (1H, m), 6.66 (1H, d, J=16.8Hz), 6.12-5.99 (1H, m), 5.28-5.20 (1H, m), 5.17-5.09 (1H, m), 4.64-4.37 (1H, m), 2.82-2.59 (2H, m), 1.94-1.66 (8H, m), 1.64 (9H, s), 1.36 (12H, s).

### Intermediate 4: Cyclopentyl (2S.4E)-2-[(tert-butoxycarbonyl)aminol-5-[3-methyl-5-(4,4,5,5-tetramethyl-1,3,2-dioxaborolan-2-yl)phenyl]pent-4-enoate

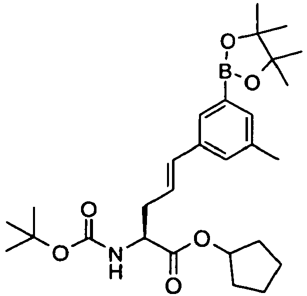

Synthesised by analogous methods to **Intermediate 2**, using **Intermediate 5** at Stage 2 of Scheme 2.
LCMS: m/z 500 [M+H]⁺. ¹H NMR (300 MHz, CDCl₃) δ: 7.60 (1H, s), 7.52 (1H, s), 7.26 (1H, s), 6.45 (1H, d, J=15.9Hz), 6.17-6.05 (1H, m), 5.27-5.20 (1H, m), 5.13-5.07 (1H, m), 4.44-4.35 (1H, m), 2.72-2.61 (2H, m), 1.93-1.64 (8H, m), 1.46 (9H,s), 1.37 (12H, s).

### Intermediate 5: 1-Bromo-3-iodo-5-methylbenzene

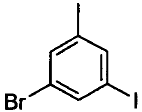

The synthesis of **Intermediate 5** is detailed within Scheme 3 and full experimental details are shown below.

### Stage 1- 4-bromo-2-iodo-6-methylaniline

To a solution of 4-bromo-2-methylaniline (1.86 g, 9.97mmol) in EtOH (33 ml) was added iodine (2.54 g, 10 mmol) and Ag₂SO₄ (3.11 g, 10 mmol). The mixture was stirred at RT for 3 hours, filtered and the solvent removed under reduced pressure. The residue was dissolved in DCM, washed with 1 M NaOH (5x20 ml) and water. The organic extracts were combined, dried (MgSO₄), filtered and concentrated *in vacuo.* The crude product was purified by column chromatography (5% EtOAc in heptane) to afford the title product as a beige solid (2.47 g, 79% yield). LCMS: m/z 312 [M+H]⁺. ¹H NMR (300 MHz, CDCl₃) δ: 7.64 (1H, s), 7.16 (1H, s), 4.10 (2H, br s), 2.20 (3H, s).

### Stage 2- 1-bromo-3-iodo-5-methylbenzene

To a solution of 4-bromo-2-iodo-6-methylaniline (2.45 g, 7.85 mmol) in a 1:3 mixture of toluene/EtOH (15 ml) at 0°C was added conc. H₂SO₄ and sodium nitrite (1.16 g, 17.28 mmol). The reaction was stirred at RT for 45 minutes then heated to reflux for 1.5 hours. The reaction was cooled to RT, diluted with water and extracted with Et₂O. The organic layer was separated and washed with water, brine, dried (MgSO₄), filtered and concentrated *in vacuo.* The crude product was purified by column chromatography (10% EtOAc in heptane) to afford the desired product as a clear oil (1.06g, 45% yield). LCMS: m/z 297 [M+H]⁺. ¹H NMR (300 MHz, CDCl₃) δ: 7.68 (1H, s), 7.49 (1H,s), 7.31 (1H, s), 2.31 (3H, s).

### Intermediate 6: 4-Bromo-2-iodo-1-methylbenzene

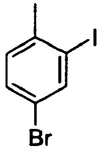

The synthesis of **Intermediates 6** is shown in Scheme 4 and full experimental details are shown below.

### Stage 1- 4-bromo-2-iodo-1-methylbenzene

To a solution of 2-methyl-5-bromo aniline (1.5 g, 8.06 mmol) in di-iodo methane (3.4 ml) at 0°C was slowly added tert-butyl nitrite (1.6 ml, 12.1 mmol). The ice bath was removed and the reaction was stirred at RT then heated at 80°C for 30 minutes. The solvent was removed under reduced pressure and the residue purified by column chromatography (EtOAc/ Heptane) to afford the title compound (1.6 g, 67% yield). LCMS: m/z 297 [M+H]⁺. ¹H NMR (300 MHz, CDCl₃) δ: 7.96 (1H, s), 7.42-7.35 (1H, m), 7.12 (1H, d, J=8.1Hz), 2.40 (3H, s).

### Intermediate 7: Cyclopentyl N-(tert-butoxycarbonyl)-O-[3-(4,4,5,5-tetramethyl-1,3,2,dioxaborolan-2-yl)phenyl]-L-homoserinate

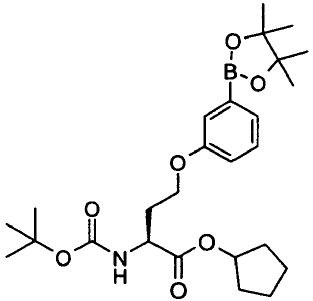

The synthesis of **intermediate 7** is detailed within Scheme 5 and full experimental details are shown below.

### Stage 1-O-[tert-Butyl(dimethyl)silyl]-L-homoserine

To a suspension of L-homoserine (1 g, 8.4 mmol) in acetonitrile (10 ml) at 0 °C was added 1,8-diazabicyclo[5.4.0]undec-7-ene (1.32 ml, 8.8 mmol). *tert*-Butyldimethylsilyl chloride (1.33 g, 8.8 mmol) was then added portionwise over 5 minutes and the reaction mixture allowed to warm to room temperature and stirred for 16 hrs. A white precipitate had formed which was filtered off and washed with acetonitrile before drying under vacuum. The title compound was isolated as a white solid (1.8 g, 92% yield). ¹H NMR (400 MHz, DMSO), δ: 7.5 (1H, br s), 3.7 (1 H, m), 3.35 (4H, bm), 1.95 (1H, m), 1.70 (1H, m), 0.9 (9 H, s), 0.1 (6H, s).

### Stage 2- N-(tert-Butoxycarbonyl)-O-[tert-butyl(dimethyl)silyl]-L-homoserine

The suspension of *O*-[*tert*-Butyl(dimethyl)silyl]-L-homoserine (1.8 g, 7.7 mmol) in DCM (100 ml) at 0°C was treated with triethylamine (2.15 ml, 15.4 mmol) and di-tert-butyl dicarbonate (1.77 g, 8.1 mmol). The reaction mixture was stirred at room temperature for 16 hours for complete reaction. The DCM was removed under reduced pressure and the mixture was treated with ethyl acetate/brine. The ethyl acetate layer was dried over magnesium sulphate and evaporated under reduced pressure. The crude product was taken forward without further purification (2.53 g, 99% yield). ¹H NMR (400 MHz, CDCl₃), δ: 7.5 (1H, br s), 5.85 (1H, d, J=6.5Hz), 4.3 (1H, m), 3.75 (2H, m), 1.95 (2H, m), 1.40 (9H, s), 0.85 (9H, s), 0.1 (6H, s).

### Stage 3- Cyclopentyl N-(tert-butoxycarbonyl)-O-[tert-butyl(dimethyl)silyl]-L-homoserinate

To a solution of *N*-(*tert*-butoxycarbonyl)-O-[*tert*-butyl(dimethyl)silyl]-L-homoserine (2.53 g, 7.6 mmol) in DCM (50 ml) at 0°C was added cyclopentanol (1.39 ml, 15.3 ml), EDCI (1.61 g, 8.4 mmol) and DMAP (0.093 g, 0.76 mmol). The reaction mixture was stirred for 16 hours at room temperature before evaporation under reduced pressure. The crude residue was dissolved in ethyl acetate (100 ml) and washed with 1 M HCl, 1 M Na₂CO₃ and brine. The organic layer was then dried over magnesium sulphate and evaporated under reduced pressure. The product was purified by column chromatography using ethyl acetate/heptane (1:4) to give 2.24 g, 73% yield of title compound. LCMS: m/z 402.5 [M+H]⁺, ¹H NMR (400 MHz, CDCl₃), δ: 5.2 (1H, d, J=6.3Hz), 5.15 (1H, m), 4.2 (1H, m), 3.6 (2H, m), 2.0 (1H, m), 1.95-1.55 (9H, bm), 1.4 (9H, s), 0.85 (9H, s), 0.1 (6H, s).

### Stage 4- Cyclopentyl (2S)-4-hydroxy-2-[(tert-butoxycarbonyl)amino]butanoate

Cyclopentyl *N*-(*tert*-butoxycarbonyl)-*O*-[*tert*-butyl(dimethyl)silyl]-L-homoserinate (1.57 g, 3.9 mmol) was dissolved in acetic acid:THF:water (3:1:1, 100 ml). The reaction mixture was stirred at 30°C for 16 hours for complete reaction. Ethyl acetate (200 ml) was added and washed with 1 M Na₂CO₃, 1 M HCl and brine. The ethyl acetate extracts were dried over magnesium sulphate and evaporated under reduced pressure to give the product as a clear oil which crystallised on standing (1.0 g, 95% yield). LCMS: m/z 310.3 [M+Na]⁺, ¹H NMR (400 MHz, CDCl3₎, δ: 5.4 (1H, d, J=6.5Hz), 5.2 (1H, m), 4.4 (1H, m), 3.65 (2H, m), 2.15 (1H, m), 1.9-1.55 (9H, bm), 1.45 (9H, s).

### Stage 5- Cyclopentyl (2S)-4-bromo-2-[(tert-butoxycarbonyl)amino]butanoate

To a slurry of *N*-bromo succinimide (1.86 g, 10.4 mmol) in DCM (16.2 ml) was added a solution of triphenyl phosphine (2.56 g, 9.74 mmol) in DCM (7.2 ml). The solution was stirred for a further 5 minutes after addition. Pyridine (338 µl, 4.18 mmol) was added, followed by a solution of cyclopentyl (2*S*)-4-hydroxy-2-[(tert-butoxycarbonyl)amino]butanoate (1.0 g, 3.48 mmol) in DCM (8.8 ml). The solution was stirred for 18 hrs, concentrated *in vacuo* and the residual solvent azeotroped with toluene (3 × 16 ml). The residue was triturated with diethyl ether (10 ml) and ethyl acetate:heptane (1:9, 2 x 10 ml). The combined ether and heptane solutions was concentrated onto silica and purified by column chromatography using 10-20% ethyl acetate in heptane to provide 1.02 g (84% yield) of title compound.
¹H NMR (400 MHz, CDCl₃), δ: 5.3-5.05 (2 H, m), 4.45-4.3 (1H, m), 3.45 (2H, t, J=7.3Hz), 2.50-2.30 (1H, m), 2.25-2.10 (1H, m), 1.95-1.60 (8H, b m), 1.47 (9H, s).

### Stage 6- Cyclopentyl O-(4-bromophenyl)-L-homoserinate

A solution of 3-bromophenol (0.593 g, 3.43 mmol) in DMF (5 ml) was cooled to 0°C with an ice bath and NaH (0.137 g, 3.43 mmol) was added in a single portion. The reaction was allowed to warm to RT, sonicated briefly and cooled down again to 0°C. A solution of cyclopentyl (2*S*)-4-bromo-2-[(tert-butoxycarbonyl)amino]butanoate (1.2 g, 3.43 mmol) in THF (10 ml) was then added dropwise, and the reaction allowed to warm to room temperature. After 1 hr the reaction was heated to 50°C and monitored by TLC for complete reaction. After 4 hrs, the reaction appeared to be complete and was allowed to cool to room temperature and poured onto a mixture of EtOAc and saturated NaHCO₃. The organic layer was collected, washed with 3 portions of water, brine and then dried (MgSO₄), filtered and evaporated *in vacuo.* The residue still contained small amounts of 4-bromophenol which was removed by scavenging with MP-carbonate (2 g) in DCM (7 ml), and the filtrate was evaporated to give the product as a white solid (1.2 g, 79% yield). LCMS: m/z 443 [M+H]⁺.

### Stage 7- Cyclopentyl N-(tert-butoxycarbonyl)-O-[4-(4,4,5,5-tetramethyl-1,3,2-dioxaborolan-2-yl)phenyl]-L-homoserinate

A mixture of cyclopentyl *O*-(4-bromophenyl)-L-homoserinate (1.2 g, 2.71 mmol), KOAc (0.346 g, 3.53 mmol), Bis[pinacolato]diboron (1.378 g, 5.43 mmol) and PdCl₂(dppf) (0.198 g, 0.271 mmol) in DMSO was heated under a nitrogen atmosphere at 65°C and monitored by LCMS for the formation of the product. After 1 hr the reaction was complete, hence the reaction mixture was cooled to room temperature and poured onto a mixture of EtOAc and 1 M HCl. The layers were separated, the organic layer washed with water and brine, dried over magnesium sulphate, filtered and evaporated under reduced pressure. The residue was purified by column chromatography eluting with 5-10% EtOAc in hexane (0.65 g, 49% yield). LCMS: m/z 490 [M+H]⁺.

### Intermediate 8: Cyclopentyl (2S,4E)-2-[(tert-butoxycarbonyl)amino]-5-{3-[4-carbamoyl-5-(carbamoylamino)-2-thienyl]phenyl}pent-4-enoate

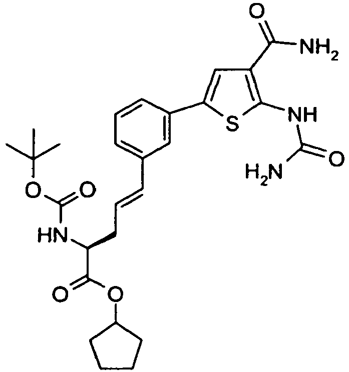

The synthesis of **Intermediate 8** follows the synthetic route shown in Scheme 1.

### Stage 5- Cyclopentyl (2S,4E)-2-[(tert-butoxycarbonyl)amino]-5-{3-[4-carbamoyl-5-(carbamoylamino)-2-thienyl]phenyl}pent-4-enoate (Intermediate 8)

To a mixture of cyclopentyl (2*S*,4*E*)-2-[(*tert*-butoxycarbonyl)amino]-5-[3-(4,4,5,5-tetramethyl-1,3,2-dioxaborolan-2-yl)phenyl]pent-4-enoate (**Intermediate 2**) (1.00, 2.06 mmol), **Intermediate 1** (1.09 g, 4.12mmol) in DME (6 ml) was added Pd(Ph₃P)₄ and sat NaHCO₃ (3ml). The mixture was purged with N₂ and heated at 75°C for 22 hrs. Reaction was cooled to RT, diluted with EtOAc, washed with H₂O, the mixture was filtered through Celite®, washed with brine, dried (MgSO₄) and concentrated under reduced pressure. The residue was purified by column chromatography (2-3.5% MeOH/DCM), then purified by preparative HPLC to provide the title compound as a solid (220 mg, 18% yield).
LCMS: m/z 565.0 [M+Na+H]⁺. ¹H NMR (300 MHz, CDCl₃) δ: 11.16 (1H, br s), 7.43 (1H, br s), 7.36-7.30 (1H, m), 7.26-7.08 (3H, m), 6.41 (1H d, J=15.9Hz), 6.24-6.05 (1H, m), 5.84-5.60 (2H, m), 5.47 (1H, d, J=8.1Hz), 5.26-5.18 (1H, m), 4.48-4.37 (1H, m), 2.77-2.54 (2H, m), 1.93-1.52 (8H, m), 1.44 (9H, s).

### Intermediates: 9a-9b

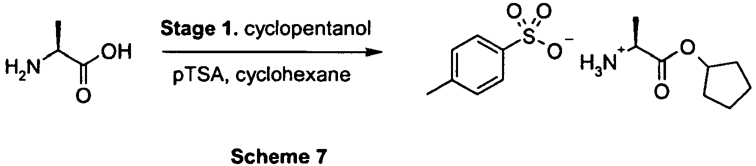

### Stage 1-(2S)-1-(cyclopentyloxy)-1-oxopropan-2-aminium 4-methylbenzenesulfonate (Intermediate 9a)

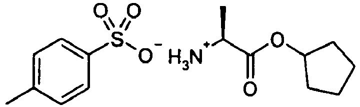

To a slurry of (S)-alanine (5 g, 55.5 mmol) in cyclohexane (150 ml) was added cyclopentanol (50 ml, 555 mmol) and p-toluene sulfonic acid (11.5 g, 55.5 mmol). The reaction was fitted with a Dean-Stark receiver and heated to 135°C for complete dissolution. After 12 hr, the reaction was cooled to RT leading to the precipitation of a white solid. The solid was filtered and washed with EtOAc before drying under reduced pressure to give the required product as a white powder (7.45 g, 85%). LCMS: m/z 159 [M+H]⁺.

### Intermediate 9b is commercially available.

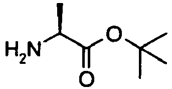

### Intermediate 10a: Cyclopentyl N-[3-(dihydroxyboryl)benzyl]-L-alaninate

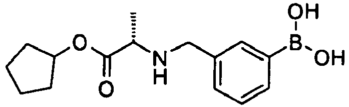

The synthesis of **Intermediate 10a** follows the synthetic route shown in Scheme 6.

To a solution of **Intermediate 9a** (500 mg, 1.51 mmol) and (3-formylphenyl) boronic acid (387 mg, 2.58 mmol) in DCM (10 ml) was added NaBH(OAc)₃ (1.63 mg, 7.69 mmol) in portions over 20 minutes. The reaction was stirred at RT for 2 hrs after which time the reaction mixture was poured into 1 M HCl (50 ml) and was washed with DCM (50 ml). The aqueous phase was neutralised to pH 7 with NaHCO₃ and extracted with DCM (2 x 50 ml). The combined organic extracts were dried over magnesium sulphate and the solvent removed. The product (650 mg, 2.23 mmol, 86% yield) was isolated and used directly.
LCMS: m/z 292 [M+H]⁺.

### Intermediate 10b: tert-Butyl N-[3-(dihydroxyboryl)benzyl]-L-alaninate

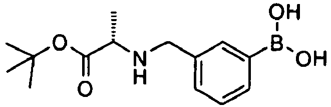

**Intermediate 10b** was prepared following the method described in Scheme 6 from **Intermediate 9b**.

### Example 1: Cyclopentyl (2S,4E)-2-amino-5-{3-[4-carbamoyl-5-(carbamoylamino)-2-thienyl]phenyl}pent-4-enoate

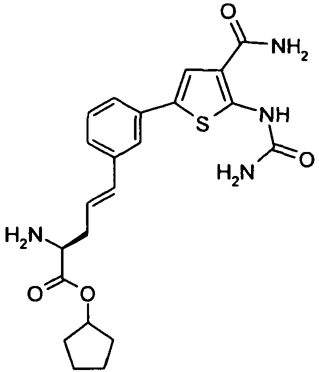

The synthesis of **Example 1** is detailed within Scheme 1.

### Stage 6- Cyclopentyl (2S,4E)-2-amino-5-{3-[4-carbamoyl-5-(carbamoylamino)-2-thienyl]phenyl}pent-4-enoate

A solution of cyclopentyl (2*S*,4*E*)-2-[(tert-butoxycarbonyl)amino]-5-{3-[4-carbamoy)-5-(carbamoylamino)-2-thienyl]phenyl}pent-4-enoate (**Intermediate 8**) (0.22 g, 0.41 mmol) in THF (2 ml) was cooled to 0°C. 4M HCl in dioxane (2.195 ml, 8.78 mmol) was added and the solution allowed to warm to room temperature with stirring. After 10 minutes, a further equivalent of HCl in dioxane (2.2 ml, 8.78 mmol) was added. The reaction was complete after 2 hours and the solvents were evaporated under reduced pressure and the residue triturated with THF (10 ml). The solid was collected, washed with a large volume of diethyl ether and dried under vacuum overnight (150 mg, 85% yield). LCMS: m/z 443.0 [M+H]⁺. ¹H NMR (300 MHz, DMSO) δ: 11.01 (1H, s), 7.76 (1H, s), 7.71 (1H, brs), 7.50 (1H, s), 7.40-7.30 (3H, m), 7.24 (1H, d, J=7.2Hz), 7.01 (2H, br s), 6.47 (1H, d, J=15.9Hz), 6.37-6.25 (1H, m), 5.10-5.04 (1H, m), 3.44 (1H, t, J=6.5Hz), 2.48-2.41 (2H, m), 1.85- 1.40 (8H, m).

The synthesis of **Example 2** is described in Scheme 1 and the experimental procedures are shown below.

### Example 2: Cyclopentyl 5-{3-[4-carbamoyl-5-(carbamoylamino)-2-thienyl]phenyl}-L-norvalinate

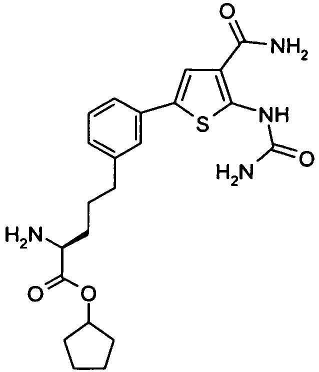

### Stage 8- Cyclopentyl 5-{3-[4-carbamoyl-5-(carbamoylamino)-2-thienyl]phenyl}-L-norvalinate

A solution of cyclopentyl (2*S*, 4*E*)-2-amino-5-{3-[4-carbamoyl-5-(carbamoylamino)-2-thienyl]phenyl}pent-4-enoate (110 mg, 0.25 mmol) in 1% acetic acid/MeOH (49 ml) was hydrogenated on the H-cube using 10% Pd/C at 1ml/min. The solution was concentrated *in vacuo* to provide the title compound as a white solid (110 mg, 90% yield). LCMS: m/z 445.1 [M+H]⁺. ¹H NMR (300 MHz, DMSO) δ: 11.01 (1H, s), 7.71 (2H, s), 7.36-6.90 (7H, m), 5.09-5.01 (1H, m), 3.33-3.25 (1H, m), 2.66-2.53 (2H, m), 1.91 (3H, s), 1.85-1.70 (2H, m), 1.68-1.39 (10H, m).

The following examples were prepared in a similar manner to **Example 1**, using **Intermediate 1** and the appropriate boronic ester, following the route shown in Scheme 1.

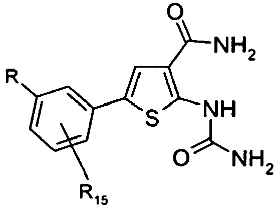

| **Example Number** | **Intermediate used** | **R** | **R₁₅** | **Name** | **LCMS purity** |
|---|---|---|---|---|---|
| **3** | **4** | 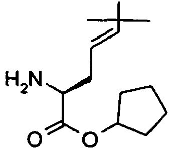 | 2-methyl | Cyclopentyl (2*S*,4*E*)-2-amino-5-{3-[4-carbamoyl-5-(carbamoylamino)-2-thienyl]-5-methylphenyl}pent-4-enoate | 99% purity: m/z 457 [M+H]+ |
| **4** | **3** | 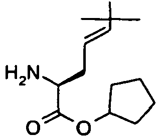 | 3-methyl | Cyclopentyl (2*S*,4*E*)-2-amino-5-{5-[4-carbamoyl-5-(carbamoylamino)-2-thienyl]-2-methylphenyl}pent-4-enoate | 99% purity: m/z 457 [M+H]+ |
| **5** | **7** | 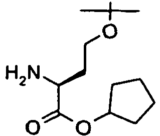 | H | Cyclopentyl O-{3-[4-carbamoyl-5-(carbamoylamino)-2-thienyl]phenyl}-L-homoserinate | 95% purity m/z 474 [M+H]+ |

### Example 6: (2S,4E)-2-amino-5-{3-{4-carbamoyl-5-(carbamoylamino)-2-thienyl]pheny}pent-4-enoic acid

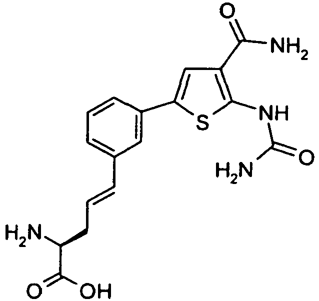

The synthetic route to **Example 6 i**s detailed in Scheme 1 using **Example 1**.

### Stage 7- (2S,4E)-2-amino-5-{3-[4-carbamoyl-5-(carbamoylamino)-2-thienyl]phenyl}pent-4-enoic acid

To a solution of cyclopentyl (2*S*, 4*E*)-2-amino-5-13-[4-carbamoyl-5 (carbamoylamino)-2-thienyl]phenyl}pent-4-enoate (**Example 1**) (0.05 g, 0.113 mmol) in THF was added lithium hydroxide (25 mg, 1.035 mmol) in 1 ml water. The solution was allowed to stir at room temperature and monitored by LCMS for the formation of the product. After 2 hrs the reaction went to completion. The THF was removed *in vacuo* and the aqueous diluted with further water (2 ml). Acetic acid was added dropwise until the pH was acidic, the precipitate formed was collected *via* filtration and washed with water (3 ml), ethanol (5 ml) and diethyl ether (10 ml). The product was isolated as an off-white solid (29 mg, 69% yield).

The following examples were prepared in a similar manner to **Example 6**.

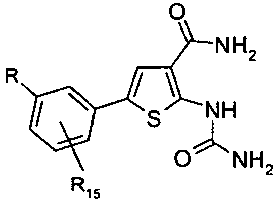

| **Example Number** | **Example used** | **R** | **R₁₅** | **Name** | **LCMS purity** |
|---|---|---|---|---|---|
| **7** | **2** | 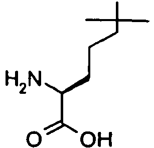 | H | 5-{3-[4-carbamoyl-5-(carbamoylamino)-2-thienyl]phenyl}-L-norvaline | 98% purity: m/z 377 [M+H]⁺ |
| **8** | **3** | 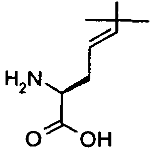 | 2-methyl | (2*S*,4*E*)-2-amino-5-{3-[4-carbamoyl-5-(carbamoylamino)-2-thienyl]-5-methylphenyl}pent-4-enoic acid | 99% purity: m/z 389 [M+H]+ |
| 9 | 4 | 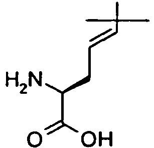 | 3-methyl | (2*S*,4*E*)-2-amino-5-{5-[4-carbamoyl-5-(carbamoylamino)-2-thienyl]-2-methylphenyl}pent-4-enoic acid | 99% purity: m/z 389 [M+H]+ |
| 10 | 5 | 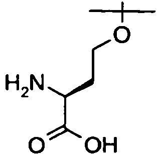 | H | *O*-{3-[4-carbamoyl-5-(carbamoylamino)-2-thienyl]phenyl}-L-homoserine | 96% purity: m/z 379 [M+H]+ |

**NMR data**

| **Example Number** | **NMR assignment** |
|---|---|
| **1** | ¹H NMR (400 MHz, DMSO- *d₆*), δ: 11.15 (1H, s), 7.90 (1H, s), 7.84 (1H, s), 7.65 (1H, s), 7.53-7.35 (4H, m), 7.14 (2H, br s), 6.60 (1H, d,J=15.9Hz), 6.49-6.40 (1H,m), 5.30-5.20 (1H, m), 3.70-3.50 (1H, m), 2.35-2.10 (2H, br s), 2.00-1.55 (8H,m). |
| **2** | ¹H NMR (400 MHz, DMSO-*d₆*), δ: 11.01 (1H, s), 7.71 (2H, s), 7.36-6.90 (7H, m), 5.09-5.01 (1H, m), 3.33-3.25 (1H, m), 2.66-2.53 (2H, m), 1.91 (3H, s), 1.85-1.70 (2H, m), 1.68-1.39 (10H, m). |
| **3** | ¹H NMR (400 MHz, DMSO- *d₆*), δ: 11.00 (1H, s), 7.74 (1H, s), 7.70 (1H, br s), 7.33 (1H, br s), 7.30 (1H, s), 7.21 (1H, s), 7.07 (1H, s), 7.00 (2H, br s), 6.41 (1H, d,J=15.9Hz), 6.34-6.22 (1H, m), 5.10-5.04 (1H,m), 3.43 (1H, t,J=6.2Hz), 2.47-2.38 (2H, m), 2.32 (3H, s), 1.85-1.40 (8H, m). |
| **4** | ¹H NMR (400 MHz, DMSO- *d₆*), δ: 10.99 (1H, s), 7.70 (2H, br s), 7.53 (1H, s), 7.5-7.27 (2H, m), 7.18 (1H, d,J=7.8Hz), 6.99 (2H, br s), 6.25-6.12 (1H, m), 5.12-5.02 (1H, m), 3.46 (1H, t, J=6.3Hz), 2.27 (3H, s), 1.85-1.45 (8H, m). |
| **5** | ¹H NMR (400 MHz, DMSO-*d₆*), δ: 11.00 (1H, br s), 7.76 (1H, s), 7.69 (1H, br s), 7.38-7.24 (2H, m), 7.11-6.91 (4H, m), 6.84-6.78 (1H, m), 5.13-5.05 (1H, m), 4.20-4.06 (2H, m), 3.50-3.43 (1H, m), 2.10-1.73 (6H, m), 1.69-1.46 (6H, m) |
| **6** | ¹H NMR (400 MHz, DMSO-*d₆*), δ: 11.02 (1H, s), 7.77 (2H, s), 7.52 (1H, s), 7.43-7.20 (4H, m), 6.99 (2H, br s), 6.50 (1H, d,J=15.9Hz), 6.37-6.25 (1H, m), 3.33-3.27 (1H, m), 2.80-2.65 (1H, m), 2.61-2.50 (1H, m). |
| **7** | ¹H NMR (400 MHz, DMSO- *d₆*), δ: 11.01 (1H, s), 7.87-7.76 (2H, m), 7.41-7.24 (2H, m), 7.09-6.92 (2H, m), 3.20-3.12 (1H, m), 2.63-2.54 (2H, m), 1.83-1.55 (4H, m). |
| **8** | ¹H NMR (400 MHz, DMSO- *d₆*), δ: 11.01 (1H, s), 7.75 (2H, s), 7.61 (2H, br s), 7.33 (2H, s), 7.22 (1H, s), 7.08 (1H, s), 7.01 (2H, br s), 6.45 (1H, d,J=15.6Hz), 6.34-6.22 (1H, m), 3.29-3.23 (1H, m), 2.76-2.65 (1H, m), 2.59-2.50 (1H, m), 2.32 (3H, s). |
| **9** | ¹H NMR (400 MHz, DMSO- *d₆*), δ: 11.00 (1H, s), 7.79 (1H, br s), 7.71 (1H, br s), 7.58 (1H, br, s), 7.32-7.28 (2H, m), 7.16 (1H, d, J=8.1Hz), 6.99 (2H, br s), 6.67 (1H, d, J=15.9Hz), 6.24-6.13 (1H, m), 3.33-3.18 (1H, m), 2.81-2.72 (1H, m), 2.62-2.53 (1H, m), 2.28 (3H, s). |
| **10** | ¹H NMR (400 MHz, DMSO- *d₆*), δ: 11.00 (1H, br s), 7.37-7.24 (2H, m), 7.14-6.90 (4H, m), 6.83-6.76 (1H, m), 4.25-4.10 (2H, m), 2.31-2.16 (1H, m), 2.06-1.93 (1H, m). |

### Example 11: Cyclopentyl N-{3[r4-carbamoyl-5-(carbamoylamino)-2-thienyl]benzyl}-L-alaninate

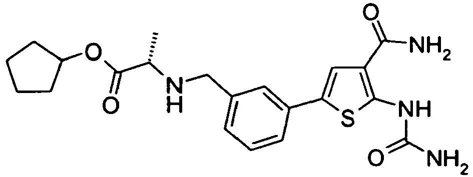

The synthesis of **Examples 11-13** is described in Scheme 6 and full experimental details are shown below.

To a solution of 5-Bromo-2-(carbamoylamino)thiophene-3-carboxamide (**Intermediate 1**) (66 mg, 0.25 mmol) in toluene/ ethanol (1:1, 4 ml) was added KOAc (50 mg, 0.5 mmol), Pd(PPh₄)₃ (15 mg, 0.013 mmol) and cyclopentyl *N*-[3-(dihydroxyboryl)benzyl]-L-alaninate (**Intermediate 10a**) (87 mg, 0.30 mmol). The reaction vial was sealed and heated at 90°C overnight. The reaction was allowed to warm to RT and the solvent removed under reduced pressure and the residue purified by preparative HPLC followed by further purification by SCX 2 resin (NH₃/MeOH) to afford the title compound (5 mg, 5% yield).
LCMS: m/z 431 [M+H]⁺. ¹H NMR (300MHz, *d*₆-DMSO) δ: 11.01 (1H, s), 7.74 (2H, br s), 7.49 (1H, s), 7.42 (1H, d, J=7.8Hz), 7.37-7.31 (2H, m), 7.18 (1H, d, J=7.8Hz), 7.01 (2H, br s), 5.16-5.06 (1H, m), 3.85-3.60 (2H, m),1.90-1.45 (8H, m), 1.23 (3H, d, J=6.9Hz).

### Example 12: tert-Butyl N-{3-[4-carbamoyl-5-(carbamoylamino)-2-thienyl]benzyl}-L-alaninate

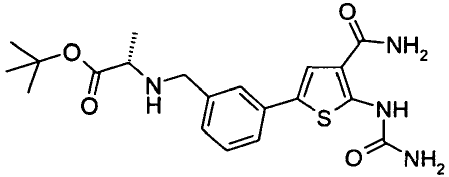

From **Intermediate 1** and **Intermediate 10b** in a similar manner to **Example 11**.

LCMS: m/z 419 [M+H]⁺. ¹H NMR (300MHz, *d*₆-DMSO) δ: 11.01 (1H, s), 7.73 (2H, br s), 7.47 (1H, s), 7.40 (1H, d, J=7.8Hz), 7.37-7.30 (2H, m), 7.17 (1H, d, J=7.2Hz), 7.00 (2H, br s), 3.75 (1H, d, J=13.5Hz), 3.60 (1H, d, J=13.5Hz), 3.20-3.10 (2H, m), 1.43 (9H, s), 1.18 (3H, d, J=6.9Hz).

### Example 13: N-{3-[4-carbamoyl-5-(carbamoylamino)-2-thienyl]benzyl}-L-alanine

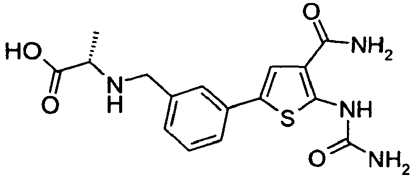

### Stage 3: N-{3-[4-carbamoyl-5-(carbamoylamino)-2-thienyl]benzyl}-L-alanine

A solution of *tert*-butyl *N*-{3-[4-carbamoyl-5-(carbamoylamino)-2-thienyl]benzyl}-L-alaninate (**Example 12**) (100 mg, 0.24 mmol) in DCM/ TFA (1:1, 5 ml) was stirred at RT overnight. The solvent was removed *in vacuo* to afford the title compound (64mg, 73% yield).
LCMS: m/z 363 [M+H]⁺. ¹H NMR (300MHz, *d*₆-DMSO) 6: 11.04 (1H, s), 7.81-7.70 (2H, m), 7.66 (1H, s), 7.60 (1H, d, J=7.9Hz), 7.45 (1H, t,J=7.7), 7.37 (1H, br s), 7.31 (1H, d, J=7.8Hz), 7.24 (1H, d, J=7.2Hz), 7.19-7.10 (1H, m), 7.04 (2H, br s), 4.11 (2H,s), 3.80-3.69 (1H, m), 1.43 (3H, d, J=7.2Hz).

### Measurement of Biological Activity

### IKKβ Enzyme Assay

The ability of compounds to inhibit IKKβ kinase activity was measured in an assay performed by Invitrogen (Paisley, UK). The Z'-LYTE™ biochemical assay employs a fluorescence-based, coupled-enzyme format and is based on the differential sensitivity of phosphorylated and non-phosphorylated peptides to proteolytic cleavage. The peptide substrate is labelled with two fluorophores-one at each end-that make up a FRET pair. In the primary reaction, the kinase transfers the gamma-phosphate of ATP to a single serine or threonine residue in a synthetic FRET-peptide. In the secondary reaction, a site-specific protease recognizes and cleaves non-phosphorylated FRET-peptides. Phosphorylation of FRET-peptides suppresses cleavage by the Development Reagent. Cleavage disrupts FRET between the donor (i.e., coumarin) and acceptor (i.e. fluorescein) fluorophores on the FRET-peptide, whereas uncleaved, phosphorylated FRET-peptides maintain FRET. A radiometric method, which calculates the ratio (the Emission Ratio) of donor emission to acceptor emission after excitation of the donor fluorophore at 400nm, is used to quantitate reaction progress.

The final 10µL Kinase Reaction consists of 0.9-8.0ng IKBKB (IKKβ), 2µM Ser/Thr 05 Peptide and ATP in 50mM HEPES pH 7.5, 0.01% BRIJ-35, 10mM MgCl₂, 1mM EGTA. The assay is performed at an ATP concentration at, or close to the Km. After the 60 minute Kinase Reaction incubation at room temperature, 5µL of a 1:128 dilution of Development Reagent is added. The assay plate is incubated for a further 60 minutes at room temperature and read on a fluorescence plate reader.
Duplicate data points are generated from a 1/3 log dilution series of a stock solution of test compound in DMSO. Nine dilutions steps are made from a top concentration of 10µM, and a 'no compound' blank is included. Data is collected and analysed using XL*fit* software from IDBS. The dose response curve is curve fitted to model number 205 (sigmoidal dose-response model). From the curve generated, the concentration giving 50% inhibition is determined and reported.

### LPS-stimulation of THP-1 cells

THP-1 cells were plated in 100µl at a density of 4 x 10° cells/well in V-bottomed 96 well tissue culture treated plates and incubated at 37°C in 5% CO₂ for 16 hrs. 2hrs after the addition of the inhibitor in 100µl of tissue culture media, the cells were stimulated with LPS (*E coli* strain 005:B5, Sigma) at a final concentration of 1µg/ml and incubated at 37°C in 5% CO₂ for 6 hrs. TNF-α levels were measured from cell-free supernatants by sandwich ELISA (R&D Systems #QTA00B)

### LPS-stimulation of human whole blood

Whole blood was taken by venous puncture using heparinised vacutainers (Becton Dickinson) and diluted in an equal volume of RPMI1640 tissue culture media (Sigma). 100µl was plated in V-bottomed 96 well tissue culture treated plates. 2hrs after the addition of the inhibitor in 100µl of RPMI1640 media, the blood was stimulated with LPS (*E coli* strain 005:B5, Sigma) at a final concentration of 100ng/ml and incubated at 37°C in 5% CO₂ for 6hrs. TNF-α levels were measured from cell-free supernatants by sandwich ELISA (R&D Systems #QTA00B)

IC50 values were allocated to one of three ranges as follows:
Range A: IC50 < 500nM
Range B: 500nM < IC50 <1000nM
Range C: IC50 >1000nM

### Results Table

| **Example Number** | **Inhibitor activity versus IKKβ** | **Inhibitor activity versus THP-1 TNFα release** | **Inhibitor activity versus human whole blood TNFα release** |
|---|---|---|---|
| 1 | A | A | A |
| 2 | A | A | C |
| 3 | A | A | C |
| 4 | A | A | C |
| 5 | A | NT | C |
| 6 | A | NR | NR |
| 7 | A | NR | NR |
| 8 | A | NR | NR |
| 9 | A | NR | NR |
| 10 | A | NR | NR |
| 11 | A | B | B |
| 12 | A | A | A |
| 13 | A | NR | NR |

| | | | |
|---|---|---|---|
| "NT" indicates the compound has not yet been tested in the assay in question. "NR" indicates "Not Relevant". Examples 6-10, 13 are the resultant carboxylic acid analogues of the amino acid esters that are cleaved inside cells. When these carboxylic acids are contacted with the cells, they do not penetrate into the cells and hence do not inhibit TNF-α in this assay. | | | |

## Claims

1. A compound selected from the group consisting of:
Cyclopentyl (2*S*,4*E*)-2-amino-5-{3-[4-carbamoyl-5 (carbamoylamino)-2-thienyl]phenyl}pent-4-enoate;
Cyclopentyl 5-{3-[4-carbamoyl-5-(carbamoylamino)-2-thienyl]phenyl}-L-norvalinate;
Cyclopentyl (2*S*,4*E*)-2-amino-5-{3-[4-carbamoyl-5-(carbamoylamino)-2-thienyl]-5-methylphenyl}pent-4-enoate;
Cyclopentyl (2*S*,4*E*)-2-amino-5-{5-[4-carbamoyl-5-(carbamoylamino)-2-thienyl]-2-methylphenyl}pent-4-enoate;
Cyclopentyl *O*-{3-[4-carbamoyl-5-(carbamoylamino)-2-thienyl]phenyl}-L-homoserinate;
Cyclopentyl N-{3-[4-carbamoyl-5-(carbamoylamino)-2-thienyl]benzyl}-L-alaninate;
*tert*-Butyl *N*-{3-[4-carbamoyl-5-(carbamoylamino)-2-thienyl]benzyl}-L-alaninate;
(2*S*,4*E*)-2-amino-5-{3-[4-carbamoyl-5-(carbamoylamino)-2-thienyl]phenyl}pent-4-enoic acid;
5-{3-[4-carbamoyl-5-(carbamoylamino)-2-thienyl]phenyl}-L-norvaline;
(2*S*,4*E*)-2-amino-5-{3-[4-carbamoyl-5-(carbamoylamino)-2-thienyl]-5-methylphenyl}pent-4-enoic acid;
(2*S*,4*E*)-2-amino-5-{5-[4-carbamoyl-5-(carbamoylamino)-2-thienyl]-2-methylphenyl}pent-4-enoic acid;
*O*-{3-[4-carbamoyl-5-(carbamoylamino)-2-thienyl]phenyl}-L-homoserine;
*N*-{3-[4-carbamoyl-5-(carbamoylamino)-2-thienyl]benzyl}-L-alanine;
and pharmaceutically acceptable salts thereof.

2. A pharmaceutical composition comprising a compound as claimed in claim 1 together with one or more pharmaceutically acceptable carriers and/or excipients.

3. A compound as claimed in claim 1 for use in treating neoplastic/proliferative, immune or inflammatory disease.

4. A compound as claimed in claim 1 for use in treating cancer cell proliferation.

5. A compound as claimed in claim 1 for use in treating hepatocellular cancer or melanoma.

6. A compound as claimed in claim 1 for use in treating bowel cancer, ovarian cancer, head and neck and cervical squamous cancers, gastric or lung cancers, anaplastic oligodendrogliomas, glioblastoma multiforme or medulloblastomas.

7. A compound as claimed in claim 1 for use in treating rheumatoid arthritis, psoriasis, inflammatory bowel disease, Crohn's disease, ulcerative colitis, chronic obstructive pulmonary disease, asthma, multiple sclerosis, diabetes, atopic dermatitis, graft versus host disease, or systemic lupus erythematosus.

8. A compound as claimed in claim 1 for use in treating metabolic or neurological disease.

9. A compound as claimed in claim 1 for use in treating Type II diabetes mellitus or Alzheimers disease.

## Patentansprüche

1. Verbindung, ausgewählt aus der Gruppe, bestehend aus:
Cyclopentyl-(2S,4E)-2-amino-5-{3-[4-carbamoyl-5-(carbamoylamino)-2-thienyl]phenyl}pent-4-enoat;
Cyclopentyl-5-{3-[4-carbamoyl-5-(carbamoylamino)-2-thienyl]phenyl}-L-norvalinat;
Cyclopentyl-(2S,4E)-2-amino-5-{3-[4-carbamoyl-5-(carbamoylamino)-2-thienyl]-5-methylphenyl}pent-4-enoat;
Cyclopentyl-(2S,4E)-2-amino-5-{5-[4-carbamoyl-5-(carbamoylamino)-2-thienyl]-2-methylphenyl}pent-4-enoat;
Cyclopentyl-O-{3-[4-carbamoyl-5-(carbamoylamino)-2-thienyl]phenyl}-L-homoserinat;
Cyclopentyl-N-{3-[4-carbamoyl-5-(carbamoylamino)-2-thienyl]benzyl}-L-alaninat;
tert-Butyl-N-{3-[4-carbamoyl-5-(carbamoylamino)-2-thienyl]benzyl}-L-alaninat;
(2S,4E)-2-Amino-5-{3-[4-carbamoyl-5-(carbamoylamino)-2-thienyl]phenyl}pent-4-ensäure;
5-{3-[4-Carbamoyl-5-(carbamoylamino)-2-thienyl]phenyl}-L-norvalin;
(2S,4E)-2-Amino-5-{3-[4-carbamoyl-5-(carbamoylamino)-2-thienyl]-5-methylphenyl}pent-4-ensäure;
(2S,4E)-2-Amino-5-{5-[4-carbamoyl-5-(carbamoylamino)-2-thienyl]-2-methylphenyl}pent-4-ensäure;
O-{3-[4-Carbamoyl-5-(carbamoylamino)-2-thienyl]phenyl}-L-homoserin;
N-{3-[4-Carbamoyl-5-(carbamoylamino)-2-thienyl]benzyl}-L-alanin;
und pharmazeutisch verträglichen Salzen davon.

2. Pharmazeutische Zusammensetzung, die eine Verbindung, wie sie in Anspruch 1 beansprucht wird, zusammen mit einem oder mehreren pharmazeutisch verträglichen Trägern und/oder Exzipientien umfasst.

3. Verbindung, wie sie in Anspruch 1 beansprucht wird, zur Verwendung bei der Behandlung einer neoplastischen/proliferativen, Immun- oder Entzündungskrankheit.

4. Verbindung, wie sie in Anspruch 1 beansprucht wird, zur Verwendung bei der Behandlung von Krebszellproliferation.

5. Verbindung, wie sie in Anspruch 1 beansprucht wird, zur Verwendung bei der Behandlung von hepatozellulärem Krebs oder Melanom.

6. Verbindung, wie sie in Anspruch 1 beansprucht wird, zur Verwendung bei der Behandlung von Darmkrebs, Eierstockkrebs, Krebs des Kopfes und des Halses und Zervix-Plattenepithelkarzinom, Magen- oder Lungenkrebs, anaplastischen Oligodendrogliomen, Glioblastoma multiforme oder Medulloblastomen.

7. Verbindung, wie sie in Anspruch 1 beansprucht wird, zur Verwendung bei der Behandlung von rheumatoider Arthritis, Psoriasis, entzündlicher Darmerkrankung, Crohn'scher Krankheit, Colitis ulcerosa, chronisch-obstruktiver Lungenerkrankung, Asthma, multipler Sklerose, Diabetes, atopischer Dermatitis, Graft-versus-Host-Krankheit oder systemischem Lupus erythematodes.

8. Verbindung, wie sie in Anspruch 1 beansprucht wird, zur Verwendung bei der Behandlung einer metabolischen oder neurologischen Krankheit.

9. Verbindung, wie sie in Anspruch 1 beansprucht wird, zur Verwendung bei der Behandlung von Diabetes mellitus, Typ II, oder Alzheimer'scher Krankheit.

## Revendications

1. Composé choisi dans l'ensemble constitué par les suivants :
(2S,4E)-2-amino-5- {3-[4-carbamyl-5-(carbamyl-amino)-thién-2-yl]-phényl}-pent-4-ènoate de cyclopentyle
5-{3-[4-carbamyl-5-(carbamyl-amino)-thién-2-yl]-phényl}-L-norvalinate de cyclopentyle
(2S,4E)-2-amino-5-{3-[4-carbamyl-5-(carbamyl-amino)-thién-2-yl]-5-méthyl-phényl}-pent-4-ènoate de cyclopentyle
(2S,4E)-2-amino-5-{5-[4-carbamyl-5-(carbamyl-amino)-thién-2-yl]-2-méthyl-phényl}-pent-4-ènoate de cyclopentyle
O-{3-[4-carbamyl-5-(carbamyl-amino)-thién-2-yl]-phényl}-L-homo-sérinate de cyclopentyle
N-{3-[4-carbamyl-5-(carbamyl-amino)-thién-2-yl]-benzyl}-L-alaninate de cyclopentyle
N-{3-[4-carbamyl-5-(carbamyl-amino)-thién-2-yl]-benzyl}-L-alaninate de tertiobutyle
acide (2S,4E)-2-amino-5-{3-[4-carbamyl-5-(carbamyl-amino)-thién-2-yl]-phényl}-pent-4-ènoïque
5-{3-[4-carbamyl-5-(carbamyl-amino)-thién-2-yl]-phényl}-L-norvaline acide (2S,4E)-2-amino-5-{3-[4-carbamyl-5-(carbamyl-amino)-thién-2-yl]-5-méthyl-phényl}-pent-4-ènoïque
acide (2S,4E)-2-amino-5-{5-[4-carbamyl-5-(carbamyl-amino)-thién-2-yl]-2-méthyl-phényl}-pent-4-ènoïque
O-{3-[4-carbamyl-5-(carbamyl-amino)-thién-2-yl]-phényl}-L-homo-sérine
N-{3-[4-carbamyl-5-(carbamyl-amino)-thién-2-yl]-benzyl}-L-alanine
ainsi que leurs sels pharmacologiquement admissibles.

2. Composition pharmaceutique comprenant un composé conforme à la revendication 1, conjointement avec un ou plusieurs véhicule(s) et/ou excipient(s) pharmacologiquement admissible(s).

3. Composé conforme à la revendication 1, pour utilisation dans le traitement d'une maladie néoplasique/proliférative, d'une maladie immunitaire ou d'une maladie inflammatoire.

4. Composé conforme à la revendication 1, pour utilisation dans le traitement d'une prolifération de cellules cancéreuses.

5. Composé conforme à la revendication 1, pour utilisation dans le traitement d'un hépatome ou d'un mélanome.

6. Composé conforme à la revendication 1, pour utilisation dans le traitement d'un cancer de l'intestin, d'un cancer des ovaires, d'un carcinome épidermoïde du col utérin ou de la tête et du cou, d'un cancer de l'estomac ou des poumons, d'un oligodendrogliome anaplasique, d'un glioblastome multiforme ou d'un médulloblastome.

7. Composé conforme à la revendication 1, pour utilisation dans le traitement de la polyarthrite rhumatoïde, du psoriasis, des maladies intestinales inflammatoires, de la maladie de Crohn, d'une colite ulcéreuse, de l'asthme, d'une bronchopneumopathie chronique obstructive, de la sclérose en plaques, d'un diabète, d'une dermatite atopique, d'une réaction d'un greffon contre l'hôte, ou du lupus érythémateux aigu disséminé.

8. Composé conforme à la revendication 1, pour utilisation dans le traitement d'une maladie métabolique ou neurologique.

9. Composé conforme à la revendication 1, pour utilisation dans le traitement du diabète sucré de type II ou de la maladie d'Alzheimer.
